(19) Europäisches Patentamt
European Patent Office
Office européen des brevets

(11) EP 0 755 669 B1

(12) **FASCICULE DE BREVET EUROPEEN**

(45) Date de publication et mention de la délivrance du brevet:
**21.01.1998 Bulletin 1998/04**

(51) Int. Cl.$^6$: **A61K 7/13**

(21) Numéro de dépôt: **96401293.4**

(22) Date de dépôt: **13.06.1996**

(54) **Procédé de teinture d'oxydation en deux temps des fibres kératiniques avec un sel de manganèse et un colorant d'oxydation et kit de teinture**

Zweistufiges Verfahren zum oxidativen Färben von Keratinfasern mit einem Magansalz und einem Oxidationsfarbstoff sowie Kit zum Färben

Oxidative two-step hair dyeing process with a manganese salt and an oxidative colorant and dyeing kit

(84) Etats contractants désignés:
**AT BE CH DE ES FR GB IT LI NL SE**

(30) Priorité: **30.06.1995 FR 9507930**

(43) Date de publication de la demande:
**29.01.1997 Bulletin 1997/05**

(73) Titulaire: **L'OREAL**
**75008 Paris (FR)**

(72) Inventeurs:
- **Audousset, Marie-Pascale**
  **92600 Asnieres (FR)**
- **Samain, Henri**
  **91570 Bievres (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 335 477**
**EP-A- 0 375 977**
**EP-A- 0 634 164**
**WO-A-87/01033**
**DE-A- 4 234 887**
**GB-A- 1 153 196**
**GB-A- 2 132 642**
**GB-A- 2 180 215**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 755 669 B1

Printed by Xerox (UK) Business Services
2.15.8/3.4

## Description

L'invention a pour objet un nouveau procédé de teinture d'oxydation en deux temps et en milieu alcalin des fibres kératiniques et en particulier des fibres kératiniques humaines telles que les cheveux mettant en oeuvre, dans un premier temps, un sel de manganèse, et dans un deuxième temps, un colorant d'oxydation en milieu alcalin oxydant.

L'invention a également pour objet le dispositif à plusieurs compartiments, ou "kit de teinture", destiné à la mise en oeuvre du procédé de teinture selon l'invention.

Il est connu de teindre les fibres kératiniques et en particulier les cheveux humains avec des compositions tinctoriales contenant des précurseurs de colorants d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé d'utiliser des sels métalliques ou des oxydes métalliques pour améliorer les propriétés tinctoriales des compositions de coloration d'oxydation et accélérer la coloration.

Ainsi, dans le brevet DE 119 187, il est décrit un procédé de teinture, en milieu acide, mettant en oeuvre, de manière simultanée (un temps) ou séparée (deux temps), un activateur d'oxydation tel que l'iode, les nitrites, les bromures, les sels de métaux lourds comme par exemple les sels de fer, de cuivre, de cobalt, de nickel, de manganèse ou de chrome, un agent oxydant et un colorant d'oxydation. Ce procédé de teinture en milieu acide conduit cependant à des colorations qui ne sont pas assez puissantes.

Il a également été proposé, dans le brevet US 4 776 856, un procédé de teinture des cheveux en un seul temps, mettant en oeuvre un colorant d'oxydation, un agent oxydant et du dioxyde de manganèse, le pH de la composition appliquée sur les cheveux étant compris entre 5,9 et 6,9. Ce procédé ne conduit pas, non plus, à des colorations suffisamment puissantes.

Or, la demanderesse vient maintenant de découvrir qu'il est possible d'obtenir, de façon rapide, des colorations particulièrement puissantes, en appliquant, et de façon séparée et étalée dans le temps (deux temps), d'abord une composition A contenant au moins un sel de manganèse puis une composition B présentant un pH alcalin et résultant du mélange extemporané (juste avant emploi) d'une composition B1 contenant au moins une base d'oxydation et d'une composition B2 contenant au moins un agent oxydant.

Cette découverte est la base de la présente invention.

L'invention a donc pour objet un procédé de teinture d'oxydation en deux temps des fibres kératiniques et en particulier des fibres kératiniques humaines telles que les cheveux, caractérisé par le fait qu'on applique sur ces fibres :

- dans un premier temps, au moins une composition A contenant, dans un milieu approprié pour la teinture, au moins un sel de manganèse,
- et dans un deuxième temps, au moins une composition B présentant un pH supérieur à 7 et résultant du mélange extemporané d'une composition B1 contenant, dans un milieu approprié pour la teinture, au moins une base d'oxydation et d'une composition B2 contenant, dans un milieu approprié pour la teinture, au moins un agent oxydant.

A temps de pose comparable, les colorations obtenues selon le procédé de l'invention sont plus puissantes que celles de l'art antérieur, et présentent une excellente ténacité vis à vis des différents traitements que peuvent subir les fibres kératiniques. De plus, le procédé de teinture d'oxydation conforme à l'invention permet de teindre les fibres kératiniques de façon rapide, d'utiliser des concentrations en colorants d'oxydation qui sont plus faibles que celles utilisées habituellement et de laisser moins de colorants d'oxydation résiduels dans les fibres kératiniques.

Selon le procédé de l'invention, l'application de la composition A sur les fibres peut être séparée de l'application de la composition B sur ces mêmes fibres par une étape intermédiaire de rinçage.

Selon une forme de réalisation particulière et préférée du procédé selon l'invention, la composition A est appliquée sur les fibres kératiniques avec un temps de pose compris de préférence entre 5 secondes et cinq minutes environ et encore plus préférentiellement entre 15 et 45 secondes environ, puis après éventuellement une étape de rinçage, la composition B est appliquée sur ces fibres avec un temps de pose compris de préférence entre 30 secondes et 45 minutes environ et encore plus préférentiellement entre 3 et 30 minutes environ.

Les sels de manganèse pouvant être utilisés dans la composition A selon le procédé de teinture de l'invention n'ont pas d'activité oxydante propre et dans ces sels, le manganèse présente de préférence un degré d'oxydation égal à 2 ou à 3. On peut bien entendu utiliser un ou plusieurs sels de manganèse.

Ils sont de préférence choisis parmi les sels hydrosolubles, minéraux ou organiques du manganèse tels que le dichlorure de manganèse, le trichlorure de manganèse, les sulfates de manganèse, les nitrates de manganèse, le diacétate de manganèse, les carbonates de manganèse, les dihydrogénocarbonates de manganèse, l'acétylacétonate de manganèse, le triacétate de manganèse, le lactate de manganèse, le formate de manganèse, l'acétyl méthionate de manganèse, le gluconate de manganèse, et leurs hydrates.

Le diacétate de manganèse tétrahydrate est particulièrement préféré.

Selon l'invention, le ou les sels de manganèse sont de préférence présents à une concentration comprise entre 0,0025 et 0,25 % en poids environ d'équivalents métal par rapport au poids total de la composition A. Encore plus préférentiellement, cette concentration est comprise entre 0,025 et 0,15 % en poids environ d'équivalents métal par rapport au poids total de la composition A.

Le pH de la composition A est généralement compris entre 3 et 9 environ, il varie de préférence entre 6 et 8 environ, et encore plus préférentiellement il est égal à 7 environ.

Le pH de la composition B doit être supérieur à 7 et est de préférence compris entre 9 et 12.

Ainsi, le pH des compositions B1 et B2 est ajusté de manière telle qu'après mélange, juste avant emploi, de la composition B1 avec la composition B2 dans un rapport pondéral variant de préférence de 0,5 à 5 et encore plus préférentiellement de 1 à 3, le pH de la composition B résultante est supérieur à 7 et de préférence compris entre 9 et 12.

Le pH des compositions A, B1, B2 et B peut être ajusté à la valeur désirée au moyen d'agents alcalinisants ou éventuellement acidifiants habituellement utilisés en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (I) suivante :

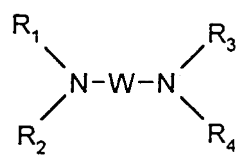

(I)

dans laquelle W est un reste propylène éventuellement substitué par un groupement hydroxyle ou un radical alkyle en $C_1$-$C_4$ ; $R_1$, $R_2$, $R_3$ et $R_4$, identiques ou différents, représentent un atome d'hydrogène, un radical alkyle en $C_1$-$C_4$ ou hydroxyalkyle en $C_1$-$C_4$.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

La nature de la ou des bases d'oxydation pouvant être utilisées dans la composition B1 selon le procédé de l'invention n'est pas critique. Cette ou ces bases d'oxydation sont de préférence choisies parmi les paraphénylènediamines, les bis-phénylalkylènediamines, les para-aminophénols, les ortho-aminophénols, les bases hétérocycliques, et leurs sels d'addition avec un acide.

Parmi les paraphénylènediamines utilisables à titre de bases d'oxydation dans la composition B1 selon le procédé de l'invention, on peut notamment citer les composés répondant à la formule (II) suivante, et leurs sels d'addition avec un acide :

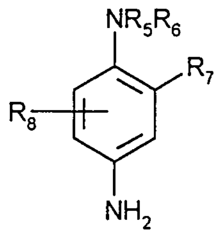

(II)

dans laquelle :

$R_5$ représente un atome d'hydrogène, un radical alkyle en $C_1$-$C_4$, monohydroxyalkyle en $C_1$-$C_4$, polyhydroxyalkyle en $C_2$-$C_4$ ou alcoxy($C_1$-$C_4$)alkyle($C_1$-$C_4$),
$R_6$ représente un atome d'hydrogène, un radical alkyle en $C_1$-$C_4$, monohydroxyalkyle en $C_1$-$C_4$ ou polyhydroxyalkyle en $C_2$-$C_4$,
$R_7$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$,
$R_8$ représente un atome d'hydrogène ou un radical alkyle en $C_1$-$C_4$.

Dans la formule (II) ci-dessus, et lorsque $R_7$ est différent d'un atome d'hydrogène, alors $R_5$ et $R_6$ représentent de préférence un atome d'hydrogène et $R_7$ est de préférence identique à $R_8$, et lorsque $R_7$ représente un atome d'halogène, alors $R_5$, $R_6$ et $R_8$ représentent de préférence un atome d'hydrogène.

Parmi les paraphénylènediamines de formule (II) ci-dessus, on peut plus particulièrement citer la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 4-amino 1-(β-méthoxyéthyl)amino benzène, la 2-chloro paraphénylènediamine, et leurs sels d'addition avec un acide.

Parmi les bis-phénylalkylènediamines utilisables à titre de bases d'oxydation dans la composition B1 selon le procédé de l'invention, on peut notamment citer les composés répondant à la formule (III) suivante, et leurs sels d'addition avec un acide :

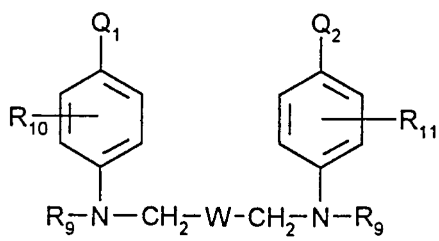

(III)

dans laquelle :

$Q_1$ et $Q_2$, identiques ou différents, représentent un radical hydroxyle ou $NHR_{12}$ dans lequel $R_{12}$ représente un atome d'hydrogène ou un radical alkyle en $C_1$-$C_4$,
$R_9$ représente un atome d'hydrogène, un radical alkyle en $C_1$-$C_4$, monohydroxyalkyle en $C_1$-$C_4$, polyhydroxyalkyle en $C_2$-$C_4$ ou aminoalkyle en $C_1$-$C_4$ dont le reste amino peut être substitué,
$R_{10}$ et $R_{11}$, identiques ou différents, représentent un atome d'hydrogène ou d'halogène ou un radical alkyle en $C_1$-$C_4$,
W représente un radical pris dans le groupe constitué par les radicaux suivants :
$-(CH_2)_n-$ ; $-(CH_2)_m$-O-$(CH_2)_m-$ ; $-(CH_2)_m$-CHOH-$(CH_2)_m-$ et

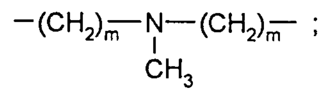

dans lesquels n est un nombre entier compris entre 0 et 8 inclusivement et m est un nombre entier compris entre 0 et 4 inclusivement.

Parmi les bis-phénylalkylènediamines de formules (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Parmi les para-aminophénols utilisables à titre de bases d'oxydation dans la composition B1 selon le procédé de l'invention, on peut notamment citer les composés répondant à la formule (IV) suivante, et leurs sels d'addition avec un acide :

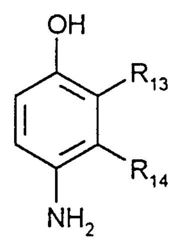

(IV)

dans laquelle :

$R_{13}$ représente un atome d'hydrogène, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$,

$R_{14}$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{13}$ ou $R_{14}$ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition B1 selon le procédé de l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition B1 selon le procédé de l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, et leurs sels d'addition avec un acide.

Selon l'invention, la ou les bases d'oxydation représentent de préférence de 0,001 à 7 % en poids environ du poids total de la composition B1 et encore plus préférentiellement de 0,01 à 2 % en poids environ de ce poids.

La composition B1 utilisée selon le procédé de l'invention peut également renfermer un ou plusieurs coupleurs

et/ou un ou plusieurs colorants directs de façon à faire varier ou enrichir en reflets les nuances obtenues avec les bases d'oxydation.

Les coupleurs utilisables dans la composition B1 mise en oeuvre dans le procédé conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents ces coupleurs représentent de préférence de 0,001 à 7 % en poids environ du poids total de la composition B1 et encore plus préférentiellement de 0,01 à 2 % en poids environ de ce poids.

Les sels d'addition avec un acide de la ou des bases d'oxydation et/ou des coupleurs utilisables dans la composition B1 de l'invention sont notamment choisis parmi les chlorhydrates, les bromhydrates, les sulfates et les tartrates, les lactates et les acétates.

L'agent oxydant présent dans la composition B2 telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. On utilise de préférence du peroxyde d'hydrogène dont la concentration est comprise entre 3 et 30 volumes (soit entre 0,9 à 9 % en poids) et encore plus préférentiellement entre 5 et 20 volumes (soit entre 1,5 à 6 % en poids).

Le milieu approprié pour la teinture (ou support) des compositions A et B1 et B2 est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther de diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de chaque compositions dans laquelle ils sont contenus, et encore plus préférentiellement entre 5 et 30 % en poids environ de ce poids.

Les compositions A et/ou B1 et/ou B2, utilisées selon le procédé conforme à l'invention, peuvent également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la mise en oeuvre selon l'invention des compositions A, B1 et B2 ne soient pas, ou substantiellement pas, altérées par la ou les adjonctions envisagées.

La composition A utilisée selon le procédé conforme à l'invention peut se présenter sous la forme d'un liquide plus ou moins épaissi et notamment sous la forme de lotion ou de shampooing.

La composition B résultant du mélange extemporané des compositions B1 et B2, utilisée selon le procédé conforme à l'invention, peut se présenter sous des formes diverses, telles que sous forme de liquides, de crèmes, de gels, de shampooings ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme une composition A contenant, dans un milieu approprié pour la teinture, au moins un sel de manganèse tel que défini précédemment, un deuxième compartiment renferme une composition B1 contenant, dans un milieu approprié pour la teinture, au moins une base d'oxydation telle que définie précédemment et un troisième compartiment renferme une composition B2 contenant, dans un milieu approprié pour la teinture au moins un agent oxydant tel que défini précédemment, le pH des compositions B1 et B2 étant ajusté de manière telle qu'après mélange de la composition B1 avec la composition B2 dans un rapport pondéral variant de préférence de 0,5 à 5 et encore plus préférentiellement de 1 à 3, le pH de la composition B résultante est supérieur à 7 et de préférence compris entre 9 et 12. Ces dispositifs peuvent être équipés d'un moyen permettant de délivrer sur les cheveux le mélange souhaité, tel que les dispositifs décrits dans le brevet FR-2 586 913

au nom de la demanderesse.

Les exemples qui suivent sont destinés à illustrer l'invention.

**EXEMPLES**

**EXEMPLE I à IV COMPARATIFS**

On a préparé les compositions I à IV suivantes (teneurs en grammes) :

| EXEMPLE | I | II (*) | III (*) | IV (*) |
|---|---|---|---|---|
| **Composition A** | **I (A)** | **II (A)** | **III (A)** | **IV (A)** |
| Acétate de manganèse, $4H_2O$ | 0,4 | - | 0,4 | - |
| Dioxyde de manganèse | - | 0,158 | - | 0,158 |
| Ethanol | 5 | 5 | 5 | 5 |
| Laurylethersulfate de sodium / magnésium 80/20 à 4 moles d'oxyde d'éthylène et en solution aqueuse à 26 % de matière active | 0,1 | 0,1 | 0,1 | 0,1 |
| Eau q.s.p. | 100 g | 100 g | 100 g | 100 g |
| **Composition B** | **I (B)** | **II (B)** | **III (B)** | **IV (B)** |
| **Composition B1** | **I (B1)** | **II (B1)** | **III (B1)** | **IV (B1)** |
| Paraphénylènediamine | 0,34 | 0,34 | 0,34 | 0,34 |
| Résorcine | 0,33 | 0,33 | 0,33 | 0,33 |
| Support de teinture commun | (**) | (**) | (**) | (**) |
| Eau q.s.p. | 100 g | 100 g | 100 g | 100 g |
| **Composition B2** | **I (B2)** | **II (B2)** | **III (B2)** | **IV (B2)** |
| $H_2O_2$ à 12,5 Volumes | 100 g | 100 g | 100 g | 100 g |
| **pH de la composition B** | **9,8** | **9,8** | **6,8 (***)** | **6,8 (***)** |

(*) : exemple ne faisant pas partie de l'invention

(***) : le pH des compositions III (B) et IV (B) a été ajusté à 6,8 avec de l'acide citrique

(**) : support de teinture commun :

| | | |
|---|---|---|
| - Octyldodécanol vendu sous la dénomination EUTANOL G par la société HENKEL | 8,0 | g |
| - Acide oléique | 20,0 | g |
| - Lauryléthersulfate de monoéthanolamine vendu sous la dénomination SIPON LM 35 par la société HENKEL | 3,0 | g |
| - Alcool éthylique | 10,0 | g |
| - Alcool benzylique | 10,0 | g |
| - Alcool cétylstéarylique à 33 moles d'oxyde d'éthylène vendu sous la dénomination SIMULSOL CS par la société SEPPIC | 2,4 | g |
| - E.D.T.A. | 0,2 | g |
| - Solution aqueuse à 60 % de matière active (M.A.) d'un polymère cationique présentant le motif récurrent suivant : 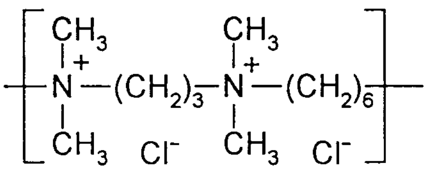 | 3,7 | g M.A. |
| - Monoéthanolamine | 7,5 | g |
| - Diéthanolamine d'acide linoléique vendu sous la dénomination COMPERLAN F par la société HENKEL | 8,0 | g |
| - Monoéthanolamine | 9,5 | g |
| - Métabisulfite de sodium en solution aqueuse à 35 % | 1,3 | g |
| - 1-phényl 3-méthyl 5-pyrazolone | 0,2 | g |

Chaque composition I(A), II(A), III(A) et IV(A) a été appliquée sur des mèches de cheveux gris permanentés à 90 % de blancs pendant 30 secondes puis les mèches ont été rincées à l'eau.

Juste avant application, chaque composition I(B1), II(B1), III(B1) et IV(B1) a été mélangée poids pour poids avec la solution (B2) de peroxyde d'hydrogène (identique pour chaque exemple), pour obtenir les compositions I(B), II(B), III(B) et IV(B).

Chaque composition (B) a ensuite été appliquée sur les mèches de cheveux pendant 5 minutes. Les cheveux ont ensuite été rincés, lavés au shampooing puis séchés.

La puissance de la coloration obtenue pour les exemples I à IV a été évaluée dans le système MUNSELL au moyen d'un colorimètre CM 2002 MINOLTA.

Selon la notation MUNSELL, une couleur est définie par l'expression H V / C dans laquelle les trois paramètres désignent respectivement la teinte ou Hue (H), l'intensité ou Value (V) et la pureté ou Chromaticité (C), la barre oblique

de cette expression est simplement une convention et n'indique pas un ratio.

La différence de couleur entre deux mèches (puissance de la coloration) a été calculée en appliquant la formule de NICKERSON :

$\Delta E = 0{,}4\ Co\Delta H + 6\Delta V + 3\ \Delta C$ , telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C, et Co représente la pureté de la mèche par rapport à laquelle on désire évaluer la différence de couleur.

Les résultats sont donnés dans le tableau ci-dessous :

| EXEMPLE | Couleur avant teinture | Couleur après teinture | Puissance de la coloration | | | |
|---|---|---|---|---|---|---|
| | | | ΔH | ΔV | ΔC | **ΔE** |
| | | | | | | |
| I | 3,7 Y 5,1 / 1,4 | 7,0 R 3,0 / 1,0 | 16,7 | 2,1 | 0,4 | **23,1** |
| II (*) | 3,7 Y 5,1 / 1,4 | 8,8 YR 4,5 / 1,3 | 4,9 | 0,6 | 0,1 | **6,6** |
| III (*) | 3,7 Y 5,1 / 1,4 | 2,3 Y 4,5 / 1,4 | 1,4 | 0,6 | 0 | **4,4** |
| IV (*) | 3,7 Y 5,1 / 1,4 | 2,1 Y 4,3 / 1,4 | 1,6 | 0,8 | 0 | **5,7** |

(*) : exemple ne faisant pas partie de l'invention

On constate que la coloration obtenue selon le procédé de l'invention (Exemple I) est beaucoup plus puissante que les colorations obtenues en mettant en oeuvre un sel de manganèse à pH acide tel que décrit par exemple dans le brevet DE 119 187 (Exemple III) ou en mettant en oeuvre du dioxyde de manganèse (exclu de l'invention), à un pH alcalin ou acide (exemples II et IV).

## Revendications

1. Procédé de teinture d'oxydation en deux temps des fibres kératiniques et en particulier des fibres kératiniques humaines telles que les cheveux, caractérisé par le fait qu'on applique sur ces fibres :

   - dans un premier temps, au moins une composition A contenant, dans un milieu approprié pour la teinture, au moins un sel de manganèse,
   - et dans un deuxième temps, au moins une composition B présentant un pH supérieur à 7 et résultant du mélange extemporané d'une composition B1 contenant, dans un milieu approprié pour la teinture, au moins une base d'oxydation et d'une composition B2 contenant, dans un milieu approprié pour la teinture, au moins un agent oxydant.

2. Procédé selon la revendication 1, caractérisé par le fait que l'application de la composition A est séparée de l'application de la composition B par une étape intermédiaire de rinçage.

3. Procédé selon la revendication 1 ou 2, caractérisé par le fait que la composition A est appliquée sur les fibres kératiniques avec un temps de pose compris entre 5 secondes et cinq minutes, puis après éventuellement une étape de rinçage, la composition B est appliquée sur les fibres avec un temps de pose compris entre 30 secondes et 45 minutes.

4. Procédé selon la revendication 3, caractérisé par le fait que la composition A est appliquée sur les fibres kératiniques avec un temps de pose compris entre 15 et 45 secondes, puis après éventuellement une étape de rinçage, la composition B est appliquée sur les fibres avec un temps de pose compris entre 3 et 30 minutes.

5. Procédé selon l'une quelconque des revendications précédentes, caractérisé par le fait que les sels de manganèse n'ont pas d'activité oxydante propre et que le manganèse présente un degré d'oxydation égal à 2 ou à 3.

6. Procédé selon la revendication 5, caractérisé par le fait que les sels de manganèse sont choisis parmi les sels

hydrosolubles, minéraux ou organiques du manganèse.

7. Procédé selon la revendication 6, caractérisé par le fait que les sels de manganèse sont choisis parmi le dichlorure de manganèse, le trichlorure de manganèse, les sulfates de manganèse, les nitrates de manganèse, le diacétate de manganèse, les carbonates de manganèse, les dihydrogénocarbonates de manganèse, l'acétylacétonate de manganèse, le triacétate de manganèse, le lactate de manganèse, le formate de manganèse, l'acétyl méthionate de manganèse, le gluconate de manganèse, et leurs hydrates.

8. Procédé selon l'une quelconque des revendications précédentes, caractérisé par le fait que le ou les sels de manganèse sont présents à une concentration comprise entre 0,0025 et 0,25 % en poids d'équivalents métal par rapport au poids total de la composition A.

9. Procédé selon la revendication 8, caractérisé par le fait que le ou les sels de manganèse sont présents à une concentration comprise entre 0,025 et 0,15 % en poids d'équivalents métal par rapport au poids total de la composition A.

10. Procédé selon l'une quelconque des revendications précédentes, caractérisé par le fait que le pH de la composition A est compris entre 3 et 9.

11. Procédé selon l'une quelconque des revendications précédentes, caractérisé par le fait que le pH de la composition B est compris entre 9 et 12.

12. Procédé selon l'une quelconque des revendications précédentes, caractérisé par le fait que la ou les bases d'oxydation présentes dans la composition B1 sont choisies parmi les paraphénylènediamines, les bis-phénylalkylènediamines, les para-aminophénols, les ortho-aminophénols, les bases hétérocycliques, et leurs sels d'addition avec un acide.

13. Procédé selon l'une quelconque des revendications précédentes, caractérisé par le fait que la ou les bases d'oxydation représentent de 0,001 à 7 % en poids du poids total de la composition B1.

14. Procédé selon la revendication 13, caractérisé par le fait que la ou les bases d'oxydation représentent de 0,01 à 2 % en poids du poids total de la composition B1.

15. Procédé selon l'une quelconque des revendications précédentes, caractérisé par le fait que la composition B1 renferme en outre un ou plusieurs coupleurs et/ou un ou plusieurs colorants directs.

16. Procédé selon l'une quelconque des revendications précédentes, caractérisé par le fait que l'agent oxydant présent dans la composition B2 est choisi le peroxyde d'hydrogène, le peroxyde d'urée, les bromates de métaux alcalins, les persels tels que les perborates et persulfates.

17. Procédé selon la revendication 16, caractérisé par le fait que l'agent oxydant est le peroxyde d'hydrogène.

18. Procédé selon l'une quelconque des revendications précédentes, caractérisé par le fait que le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

19. Dispositif à plusieurs compartiments ou "kit" de teinture à plusieurs compartiments, caractérisé par le fait qu'un premier compartiment renferme une composition A contenant, dans un milieu approprié pour la teinture, au moins un sel de manganèse tel que défini à l'une quelconque des revendications 5 à 7, un deuxième compartiment renferme une composition B1 contenant, dans un milieu approprié pour la teinture, au moins une base d'oxydation et un troisième compartiment renferme une composition B2 contenant, dans un milieu approprié pour la teinture au moins un agent oxydant, le pH des compositions B1 et B2 étant ajusté de manière telle qu'après mélange de la composition B1 avec la composition B2 dans un rapport pondéral variant de 0,5 à 5, le pH de la composition B résultante est supérieur à 7.

## Claims

1. Process for the two-stage oxidation dyeing of keratin fibres, and in particular human keratin fibres such as the hair, characterized in that the following are applied to these fibres:

   - in a first stage, at least one composition A containing, in a medium which is suitable for dyeing, at least one manganese salt,
   - and, in a second stage, at least one composition B having a pH above 7 and resulting from the extemporaneous mixing of a composition B1 containing, in a medium which is suitable for dyeing, at least one oxidation base and of a composition B2 containing, in a medium which is suitable for dyeing, at least one oxidizing agent.

2. Process according to Claim 1, characterized in that the application of the composition A is separated from the application of the composition B by an intermediate rinsing step.

3. Process according to Claim 1 or 2, characterized in that the composition A is applied to the keratin fibres over an exposure time of between 5 seconds and five minutes, then, after an optional rinsing step, the composition B is applied to the fibres over an exposure time of between 30 seconds and 45 minutes.

4. Process according to Claim 3, characterized in that the composition A is applied to the keratin fibres over an exposure time of between 15 and 45 seconds, then, after an optional rinsing step, the composition B is applied to the fibres over an exposure time of between 3 and 30 minutes.

5. Process according to any one of the preceding claims, characterized in that the manganese salts have no intrinsic oxidizing activity and in that the manganese has an oxidation state equal to 2 or 3.

6. Process according to Claim 5, characterized in that the manganese salts are chosen from inorganic or organic water-soluble manganese salts.

7. Process according to Claim 6, characterized in that the manganese salts are chosen from manganese dichloride, manganese trichloride, manganese sulphates, manganese nitrates, manganese diacetate, manganese carbonates, manganese dihydrogenocarbonates, manganese acetylacetonate, manganese triacetate, manganese lactate, manganese formate, manganese acetylmethionate and manganese gluconate, and the hydrates thereof.

8. Process according to any one of the preceding claims, characterized in that the manganese salt or salts are present at a concentration of between 0.0025 and 0.25% by weight of metal equivalents relative to the total weight of the composition A.

9. Process according to Claim 8, characterized in that the manganese salt or salts are present at a concentration of between 0.025 and 0.15% by weight of metal equivalents relative to the total weight of the composition A.

10. Process according to any one of the preceding claims, characterized in that the pH of the composition A is between 3 and 9.

11. Process according to any one of the preceding claims, characterized in that the pH of the composition B is between 9 and 12.

12. Process according to any one of the preceding claims, characterized in that the oxidation base or bases present in the composition B1 are chosen from para-phenylenediamines, bis(phenyl)alkylenediamines, para-aminophenols, ortho-aminophenols and heterocyclic bases, and the addition salts thereof with an acid.

13. Process according to any one of the preceding claims, characterized in that the oxidation base or bases represent from 0.001 to 7% by weight relative to the total weight of the composition B1.

14. Process according to Claim 13, characterized in that the oxidation base or bases represent from 0.01 to 2% by weight relative to the total weight of the composition B1.

15. Process according to any one of the preceding claims, characterized in that the composition B1 also includes one or more couplers and/or one or more direct dyes.

16. Process according to any one of the preceding claims, characterized in that the oxidizing agent present in the composition B2 is chosen from hydrogen peroxide, urea peroxide, alkali metal bromates and persalts such as perborates and persulphates.

17. Process according to Claim 16, characterized in that the oxidizing agent is hydrogen peroxide.

18. Process according to any one of the preceding claims, characterized in that the medium which is suitable for dyeing (or support) consists of water or of a mixture of water and at least one organic solvent chosen from $C_1$-$C_4$ lower alkanols, glycerol, glycols and glycol ethers, aromatic alcohols, similar products and mixtures thereof.

19. Multi-compartment device or multi-compartment dyeing "kit", characterized in that a first compartment includes a composition A containing, in a medium which is suitable for dyeing, at least one manganese salt as defined in any one of Claims 5 to 7, a second compartment includes a composition B1 containing, in a medium which is suitable for dyeing, at least one oxidation base, and a third compartment includes a composition B2 containing, in a medium which is suitable for dyeing, at least one oxidizing agent, the pH of the compositions B1 and B2 being adjusted such that after mixing the composition B1 with the composition B2 in a weight ratio ranging from 0.5 to 5, the pH of the resulting composition B is above 7.

**Patentansprüche**

1. Zweistufiges Verfahren zum oxidativen Färben von Keratinfasern und insbesondere menschlichen Keratinfasern, wie dem Haar, dadurch gekennzeichnet, daß auf die Fasern aufgetragen werden:

   - in einem ersten Schritt mindestens eine Zusammensetzung A, die in einem zum Färben geeigneten Medium mindestens ein Mangansalz enthält,

   - und in einem zweiten Schritt mindestens eine Zusammensetzung B, deren pH-Wert größer 7 ist und die hergestellt wird, indem eine Zusammensetzung B1, die in einem zum Färben geeigneten Medium mindestens eine Oxidationsbase enthält, und eine Zusammensetzung B2, die in einem zum Färben geeigneten Medium mindestens ein Oxidationsmittel enthält, unmittelbar vor der Anwendung vermischt werden.

2. Verfahren nach Anspruch 1, dadurch gekennzeichnet, daß nach dem Aufbringen der Zusammensetzung A und vor dem Aufbringen der Zusammensetzung B in einem Zwischenschritt gespült wird.

3. Verfahren nach Anspruch 1 oder 2, dadurch gekennzeichnet, daß die Zusammensetzung A mit einer Einwirkungszeit im Bereich von 5 Sekunden bis fünf Minuten auf die Keratinfasern aufgebracht wird, und daß anschließend, nachdem gegebenenfalls gespült wurde, die Zusammensetzung B mit einer Einwirkungszeit im Bereich von 30 Sekunden bis 45 Minuten auf die Fasern aufgebracht wird.

4. Verfahren nach Anspruch 3, dadurch gekennzeichnet, daß die Zusammensetzung A mit einer Einwirkungszeit im Bereich von 15 bis 45 Sekunden auf die Keratinfasern aufgebracht wird, und daß anschließend, nachdem gegebenenfalls gespült wurde, die Zusammensetzung B mit einer Einwirkungszeit im Bereich von 3 bis 30 Minuten auf die Fasern aufgebracht wird.

5. Verfahren nach einem der vorhergehenden Ansprüche, dadurch gekennzeichnet, daß die Mangansalze selbst nicht oxidierend wirken und daß das Mangan in einer Oxidationsstufe von 2 oder 3 vorliegt.

6. Verfahren nach Anspruch 5, dadurch gekennzeichnet, daß die Mangansalze unter den wasserlöslichen, anorganischen oder organischen Mangansalzen ausgewählt sind.

7. Verfahren nach Anspruch 6, dadurch gekennzeichnet, daß die Mangansalze unter Mangandichlorid, Mangantrichlorid, Mangansulfaten, Mangannitraten, Mangandiacetat, Mangancarbonaten, Mangandihydrogencarbonaten, Manganacetylacetonat, Mangantriacetat, Manganlactat, Manganformiat, Manganacetylmethionat, Mangangluconat und den Hydraten dieser Verbindungen ausgewählt sind.

8. Verfahren nach einem der vorhergehenden Ansprüche, dadurch gekennzeichnet, daß das oder die Mangansalz(e) in einer Konzentration im Bereich von 0,0025 bis 0,25 Gew.-%, ausgedrückt als Metall, vorliegen, bezogen auf das Gesamtgewicht der Zusammensetzung A.

9. Verfahren nach Anspruch 8, dadurch gekennzeichnet, daß das oder die Mangansalz(e) in einer Konzentration im Bereich von 0,025 bis 0,15 Gew.-%, ausgedrückt als Metall, vorliegen, bezogen auf das Gesamtgewicht der Zusammensetzung A.

10. Verfahren nach einem der vorhergehenden Ansprüche, dadurch gekennzeichnet, daß der pH-Wert der Zusammensetzung A im Bereich von 3 bis 9 liegt.

11. Verfahren nach einem der vorhergehenden Ansprüche, dadurch gekennzeichnet, daß der pH-Wert der Zusammensetzung B im Bereich von 9 bis 12 liegt.

12. Verfahren nach einem der vorhergehenden Ansprüche, dadurch gekennzeichnet, daß die in der Zusammensetzung B1 enthaltene(n) Oxidationsbase(n) unter den p-Phenylendiaminen, Bisphenylalkylendiaminen, p-Aminophenolen, o-Aminophenolen, heterocyclischen Basen und den Additionssalzen dieser Verbindungen mit einer Säure ausgewählt sind.

13. Verfahren nach einem der vorhergehenden Ansprüche, dadurch gekennzeichnet, daß die Oxidationsbase(n) einen Gewichtsanteil von 0,001 bis 7 Gew.-% des Gesamtgewichts der Zusammensetzung B1 ausmachen.

14. Verfahren nach Anspruch 13, dadurch gekennzeichnet, daß die Oxidationsbase(n) einen Gewichtsanteil von 0,01 bis 2 Gew.-% des Gesamtgewichts der Zusammensetzung B1 ausmachen.

15. Verfahren nach einem der vorhergehenden Ansprüche, dadurch gekennzeichnet, daß die Zusammensetzung B1 ferner einen oder mehrere Kuppler und/oder einen oder mehrere Direktfarbstoff(e) enthält.

16. Verfahren nach einem der vorhergehenden Ansprüche, dadurch gekennzeichnet, daß das in der Zusammensetzung B2 enthaltene Oxidationsmittel unter Wasserstoffperoxid, Harnstoffperoxid, Alkalimetallbromaten und Salzen von Persäuren, wie Perboraten und Persulfaten, ausgewählt ist.

17. Verfahren nach Anspruch 16, dadurch gekennzeichnet, daß das Oxidationsmittel Wasserstoffperoxid ist.

18. Verfahren nach einem der vorhergehenden Ansprüche, dadurch gekennzeichnet, daß das zum Färben geeignete Medium (oder der Träger) aus Wasser oder einem Gemisch aus Wasser und mindestens einem organischen Lösungsmittel besteht, das unter den niederen Alkanolen mit 1 bis 4 Kohlenstoffatomen, Glycerin, Glykolen und Glykolethern, aromatischen Alkoholen, analogen Produkten und deren Gemischen ausgewählt ist.

19. Vorrichtung mit mehreren Abteilungen oder 'Kit' zum Färben mit mehreren Abteilungen, dadurch gekennzeichnet, daß eine erste Abteilung eine Zusammensetzung A, die in einem zum Färben geeigneten Medium mindestens ein Mangansalz nach einem der Ansprüche 5 bis 7 enthält, eine zweite Abteilung eine Zusammensetzung B1, die in einem zum Färben geeigneten Medium mindestens eine Oxidationsbase enthält, und eine dritte Abteilung eine Zusammensetzung B2 enthält, die in einem zum Färben geeigneten Medium mindestens ein Oxidationsmittel enthält, wobei der pH-Wert der Zusammensetzungen B1 und B2 so eingestellt ist, daß nach dem Mischen der Zusammensetzung B1 mit der Zusammensetzung B2 in einem Gewichtsverhältnis, das im Bereich von 0,5 bis 5 liegt, der pH-Wert der resultierenden Zusammensetzung B größer 7 ist.